(19) **Europäisches Patentamt**
European Patent Office
Office européen des brevets

(11) **EP 2 978 362 B1**

(12) **EUROPÄISCHE PATENTSCHRIFT**

(45) Veröffentlichungstag und Bekanntmachung des Hinweises auf die Patenterteilung:
**23.09.2020 Patentblatt 2020/39**

(51) Int Cl.:
*A61B 1/12* (2006.01)  *A61B 90/70* (2016.01)

(21) Anmeldenummer: **14709882.6**

(22) Anmeldetag: **10.03.2014**

(86) Internationale Anmeldenummer:
**PCT/EP2014/000606**

(87) Internationale Veröffentlichungsnummer:
**WO 2014/154327 (02.10.2014 Gazette 2014/40)**

(54) **VERFAHREN UND SYSTEM ZUR ÜBERWACHUNG EINER AUFBEREITUNGSVORRICHTUNG FÜR ENDOSKOPE**

METHOD AND SYSTEM FOR MONITORING A REPROCESSING DEVICE FOR ENDOSCOPES

PROCÉDÉ ET SYSTÈME DE SURVEILLANCE D'UN DISPOSITIF DE TRAITEMENT POUR ENDOSCOPES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.03.2013 DE 102013205296**

(43) Veröffentlichungstag der Anmeldung:
**03.02.2016 Patentblatt 2016/05**

(73) Patentinhaber: **Olympus Winter & Ibe GmbH**
**22045 Hamburg (DE)**

(72) Erfinder:
• **CARLSON, Torben**
**22047 Hamburg (DE)**
• **THATE, Henning**
**22417 Hamburg (DE)**

(74) Vertreter: **Seemann & Partner Patentanwälte mbB**
**Raboisen 6**
**20095 Hamburg (DE)**

(56) Entgegenhaltungen:
EP-A2- 1 529 484    DE-A1- 10 135 137
US-A- 5 422 276    US-A1- 2010 071 736

**Beschreibung**

Die Erfindung betrifft ein Verfahren zur Überwachung einer Aufbereitungsvorrichtung für Endoskope, insbesondere einer Reinigungs- und/oder Desinfiziervorrichtung. Die Erfindung betrifft ferner ein entsprechendes System zur Überwachung einer Aufbereitungsvorrichtung für Endoskope, umfassend wenigstens eine Aufbereitungsvorrichtung und wenigstens eine Auswertungsvorrichtung.

An die Aufbereitung von Endoskopen nach deren Benutzung werden u. a. im klinischen Bereich hohe Anforderungen gestellt. Die Aufbereitung umfasst üblicherweise sowohl ein Waschen, ein Desinfizieren und ein Trocknen der Endoskope. Der Desinfizierung gehen üblicherweise ein oder zwei Wasch- oder Vorwaschgänge voraus, es gibt Spülgänge mit klarem Wasser und Trocknungsgänge. Zum Waschen und Desinfizieren werden jeweils Waschmittel bzw. eine oder mehrere Chemikalien zum Desinfizieren hinzudosiert. Dies erfolgt üblicherweise in automatisierten Aufbereitungsvorrichtungen.

Eine entsprechende automatische bzw. automatisierte Aufbereitungsvorrichtung wird beispielsweise von der Anmelderin unter der Hersteller- und Typenbezeichnung Olympus ETD3 vertrieben, wobei ETD für "Endo Thermo Disinfector" steht. Diese Aufbereitungsvorrichtung ist mit verschiedenen Aufbereitungsprogrammen ausgestattet und erlaubt die simultane Aufbereitung mehrerer flexibler oder starrer Endoskope. Es bietet eine Desinfektion mit der herkömmlich verwendeten Chemikalie Glutaraldehyd und alternativ auf der Grundlage von Peressigsäure (peracetic acid, PAA). Mit der PAA zusammen wird außerdem eine Aktivatorlösung hinzudosiert. Zusätzlich umfasst die ETD3 eine UV-Einheit, mit der Spülwasser weiter desinfiziert werden kann.

Die ETD3 verfügt über eine umfangreiche Sensorik und über Protokollierfunktionen. So wird zu den Aufbereitungsvorgängen jeweils neben entsprechenden Zeitstempeln unter anderem mittels Flügelrad-Durchflussmessern die Menge der verschiedenen Aufbereitungsmittel, also Wasserdosierung, Waschmitteldosierung und Chemikaliendosierung erfasst und protokolliert. Weiter verfügt die ETD3 über eine automatische transponderbasierte Erkennung kompatibler Endoskope über das EndoID-System von Olympus. Mit diesem System werden u. a. die Seriennummer der Aufbereitungsvorrichtung, Typ und Seriennummer des aufbereiteten Endoskops, Name der Bedienperson, die den Aufbereitungsvorgang gestartet hat und weitere Prozessparameter protokolliert. Das EndoID-System erlaubt es, nach Erkennen des aufzubereitenden Endoskops die Prozessparameter für das Endoskop automatisch richtig einzustellen. Die ETD3 verfügt außerdem über eine Leckerkennung.

Um gegebenenfalls vorhandenen Dokumentationspflichten nachzukommen, kann im Anschluss an den Aufbereitungsvorgang ein Report darüber generiert werden, ob alle Prozessparameter des Aufbereitungsvorgangs korrekt waren, also sich innerhalb kalibrierter oder vorgegebener Parameterbereiche befanden, und dass ein ausreichendes Aufbereitungsergebnis erzielt worden ist. Dieser Report kann ausgedruckt oder direkt, beispielsweise über eine ISDN- oder LAN-Verbindung, an ein Endoskop-Informationsmanagementsystem übermittelt werden. Außerdem bietet die ETD3 die Möglichkeit der Fernwartung.

EP 1 529 484 A2 betrifft die Analyse von Endoskopkanälen, wobei eine Flüssigkeit durch den zu überprüfenden Kanal geleitet wird und ein mit der Durchlässigkeit des Kanals verknüpfter Messwert gemessen wird. Der Kanal wird identifiziert und der Messwert in Zuordnung zu dem identifizierten Kanal gespeichert. Es wird eine Trendanalyse der Messwerte nach an sich bekannten mathematischen Methoden durchgeführt.

US 5,422,267 A betrifft ein Verfahren und eine Vorrichtung zur Sterilisierung, wobei die Vorrichtung testet, ob Luft vollständig aus einer Sterilisationskammer entfernt worden ist und ob effektive Sterilisationsbedingungen vorliegen. Es werden die Ursachen von Sterilisationsfehlern identifiziert, eine Frühwarnung ausgegeben, indem frühere Testresultate gespeichert werden und eine Trendanalyse durchgeführt wird, falls die Trendresultate einen Trend aufweisen, der auf ein zukünftiges Versagen hinweist.

Aus US 2010/0071736 A1 ist ferner eine Reinigungsvorrichtung zur gleichzeitigen Reinigung mehrerer Endoskope bekannt, bei der eine Kombinationsautorisierungsinformationsspeichereinheit Kombinationsautorisierungsinformationen speichert, welche für beliebige Kombinationen unterschiedlicher Endoskoptypen aussagen, ob diese Endoskoptypen in der jeweiligen Kombination gleichzeitig gereinigt werden dürfen oder nicht.

Ausgehend von diesem Stand der Technik liegt der vorliegenden Erfindung die Aufgabe zugrunde, die Überwachung von Aufbereitungsvorrichtungen für Endoskope und von Endoskopen zu verbessern.

Diese Aufgabe wird durch ein Verfahren zur Überwachung einer Aufbereitungsvorrichtung für Endoskope, insbesondere einer Reinigungs- und/oder Desinfiziervorrichtung, gelöst, wobei über eine Mehrzahl von Aufbereitungsvorgängen, die jeweils mehrere Teilvorgänge umfassen, von wenigstens einem Endoskop in einer einzigen Aufbereitungsvorrichtung mehrere Datenpunkte eines Prozessparameters protokolliert werden, wobei bei jedem Teilvorgang ein unterschiedlicher Bedarf des Prozessparameters besteht, wobei in jedem Datenpunkt des Prozessparameters ein Zeitpunkt des jeweiligen Aufbereitungsvorgangs protokolliert und in Zuordnung zum jeweiligen Teilvorgang gespeichert wird, wobei in der Auswertungsvorrichtung über die Teilvorgänge eine Trendanalyse der mehreren Datenpunkte durchgeführt wird, wobei aus der Trendanalyse eine IST-Kalibration des Prozessparameters errechnet wird und durch Vergleich mit einer Soll-Kalibration erkannt wird, ob eine Fehlkalibration der Aufbereitungsvorrichtung vorliegt.

Über die bisher gehandhabte Protokollierung und Dokumentationhinaus wird somit erfindungsgemäß eine weiterführende Datenanalyse mit an sich bekannten statistischen Methoden, etwa linearen oder anderen Regressionsanalysen, durchgeführt, mit der unter anderem ein entstehender Wartungsbedarf für eine Aufbereitungseinrichtung frühzeitig erkannt werden kann, so dass geeignete Maßnahmen rechtzeitig ergriffen werden können, bevor es aufgrund des Auftretens nicht spezifikationsgetreuer Prozessparameter dazu kommt, dass Aufbereitungsvorgänge nicht regel- und spezifikationskonform durchgeführt werden und es zu mangelhaften Aufbereitungsergebnissen kommt.

Unter einem Trend wird im Rahmen der vorliegenden Erfindung eine zeitliche Entwicklung verstanden, aber auch Auffälligkeiten, die von anderen Prozessparametern abhängen, beispielsweise vermehrt bei einzelnen Aufbereitungsprogrammen auftreten oder bei bestimmten Bedienpersonen. In den ersten Fällen können beispielsweise Wartungen angesetzt werden, also Wartungen vor Ort oder gegebenenfalls Fernwartungen. Im letzteren Fall kann beispielsweise der Schulungsbedarf der Mitarbeiter besser koordiniert werden.

Mit dem erfindungsgemäßen Verfahren wird auf diese Weise sowohl die Qualität der Aufbereitungsergebnisse als auch die Effizienz der Aufbereitung von Endoskopen verbessert, weil mangelhafte Aufbereitungsergebnisse bereits bei Erkennen entsprechender Trends vor ihrem Auftreten abgewendet werden können.

In vorteilhaften Ausführungsformen des Verfahrens wird oder werden ein Aufbereitungsvorrichtungsindikator, ein Bedienpersonenindikator, ein Aufbereitungsprogrammindikator, wenigstens ein Endoskopindikator für wenigstens ein aufbereitetes Endoskop, wenigstens ein Aufbereitungsmittelindikator, ein Druckverlust und/oder eine Druckverlustgeschwindigkeit, wenigstens eine Dosiermenge wenigstens eines zu dosierenden Aufbereitungsmittels, ein Start- und/oder Endzeitpunkt des Aufbereitungsvorgangs, eine Prozessdauer, Fehlfunktionen, Fehlbedienungen und/oder Fehlermeldungen protokolliert. Dabei ist ein Aufbereitungsvorrichtungsindikator beispielsweise eine Seriennummer der Aufbereitungsvorrichtung, ein Bedienpersonenindikator deren Name oder Personen-Kennnummer, ein Aufbereitungsprogrammindikator beispielsweise ein Programmname oder eine das Aufbereitungsprogramm kennzeichnende Bezeichnung oder Nummer, ein Endoskopindikator eine Typenbezeichnung und/oder eine Seriennummer eines Endoskops, ein Aufbereitungsmittelindikator beispielsweise ein Chemikalienname oder eine Chemikalienkennzeichnung. Wenn mehrere dieser Prozessparameter protokolliert und analysiert werden, erhöht dies die Prozesssicherheit, Qualität und Effizienz der Endoskopaufbereitung.

Für Aufbereitungsvorgänge, bei denen mehrere verschiedene Teilvorgänge durchgeführt werden, werden teilvorgangsrelevante Prozessparameter in Bezug auf die einzelnen Teilvorgänge protokolliert, insbesondere ein Teilvorgangsidentifikator, Dauer des Teilvorgangs und/oder Dosiermengen eines oder mehrerer während des Teilvorgangs zu dosierender Aufbereitungsmittel. Somit ist eine engmaschige und spezifische Trendanalyse möglich. Als Teilvorgänge werden beispielsweise Vorwaschgänge, Waschgänge, Desinfektionsgänge, Spülgänge, Trocknungsgänge u. a. verstanden, die jeweils mit eigenen Prozessparametern durchgeführt werden. So können wartungsrelevante Auffälligkeiten in einzelnen Teilvorgängen identifiziert und behoben werden.

In der Trendanalyse wird vorzugsweise wenigstens ein protokollierter Prozessparameter als Funktion der jeweiligen Start- oder Endzeitpunkte der Aufbereitungsvorgänge, des Aufbereitungsvorrichtungsindikators, des Aufbereitungsprogrammindikators, des Teilvorgangsindikators, des Bedienpersonenindikators und/oder des Endoskopindikators dargestellt und/oder ausgewertet.

Die zeitliche Trendanalyse betrifft beispielsweise typischerweise die Dosierung der Desinfektionschemikalie oder Desinfektionschemikalien oder anderer Aufbereitungsmittel. In einer Mehrzahl aufeinanderfolgender Aufbereitungsvorgänge kann beispielsweise die Chemikaliendosis innerhalb des erlaubten Bereichs mehr oder weniger kontinuierlich abnehmen oder ansteigen. Jede einzelne Dosierung und das damit erzielte Desinfektionsresultat sind somit spezifikationskonform und unauffällig. Ein Wartungsbedarf wurde bisher erst dann festgestellt, wenn sich dieser Trend fortgesetzt hat und die Dosierung tatsächlich aus dem regelkonformen Bereich heraus gewandert ist. Eine erfindungsgemäße Trendanalyse deckt den herrschenden Trend allerdings auf, so dass in einer Wartung Gegenmaßnahmen ergriffen werden können, bevor das Desinfektionsergebnis mangelhaft wird oder umgekehrt mehr Desinfektionschemikalien verbraucht werden als nötig.

Eine Auswertung des oder der protokollierten Prozessparameter als Funktion der anderen Indikatoren erlaubt eine Erkennung von Unregelmäßigkeiten, die nicht zeitlich abhängig auftreten, sondern für einzelne Aufbereitungsvorrichtungen, Aufbereitungsprogramme, Teilvorgänge, Bedienpersonen oder Endoskope typisch sind. In diesem Zusammenhang werden vorteilhafterweise die protokollierten Daten für die Darstellung und/oder Auswertung nach dem Zeitpunkt, dem Aufbereitungsvorrichtungsindikator, dem Bedienpersonenindikator, dem Aufbereitungsprogrammindikator, dem Teilprogrammindikator und/oder dem wenigstens einen Endoskopindikator gefiltert. So wird eine detaillierte Trendanalyse verwirklicht.

Ein Anwendungsbeispiel hierfür ist etwa die Überwachung der Kalibration von Dosiervorrichtungen in einer Aufbereitungsvorrichtung. Hierfür kann die Abweichung einer Dosiermenge von der gewünschten Dosiermenge für eine bestimmte Dosiervorrichtung in Abhängigkeit der Größe der gewünschten Dosiermenge analysiert werden. Steigt oder fällt diese Differenz mit steigender gewünschter Dosiermenge, so ist die Kalibrierung der Dosiervorrichtung anzupassen.

In der Trendanalyse wird vorzugsweise analysiert, ob sich mit der Zeit ein protokollierter Prozessparameter, insbesondere eine Dosiermenge oder eine Prozessdauer, so entwickelt, dass dieser Prozessparameter bei sich fortsetzendem Trend einen kalibrierten Bereich oder einen Toleranzbereich verlässt. Dabei wird vorteilhafterweise ein Wartungsbedarf der Aufbereitungsvorrichtung signalisiert, bevor ein protokollierter Prozessparameter den kalibrierten Bereich oder Toleranzbereich verlässt, wenn die Trendanalyse einen solchen Trend ergibt.

Ebenfalls vorteilhafterweise wird ein Schulungsbedarf einer Bedienperson signalisiert, wenn die Trendanalyse ergibt, dass bei der Bedienperson die Häufigkeit des Auftretens von Fehlfunktionen, Fehlbedienungen und/oder Fehlermeldungen gegenüber anderen Bedienpersonen erhöht ist.

Auch ein Druckverlust und/oder eine Druckverlustgeschwindigkeit ist ein wichtiger, der Trendanalyse zugänglicher, Prozessparameter. In einigen Aufbereitungsvorrichtungen wird vor Beginn der Endoskopaufbereitung eine Dichtigkeitsprüfung der Endoskope durchgeführt. Hierzu wird ein geringer Überdruck erzeugt und der Druckabfall in einem bestimmten Zeitraum geprüft. Ist der Druckverlust oberhalb einer definierten Schwelle eines geringen und zulässigen Druckverlustes, so wird das Endoskop als undicht gemeldet und die Wiederaufbereitung nicht gestartet. Damit wird verhindert, dass während der Aufbereitung Feuchtigkeit in sensible Teile des Endoskops gelangt. Ohne Trendanalyse wird eine Undichtigkeit immer dem Endoskop zugeordnet und werden somit Undichtigkeiten im Bereich der Aufbereitungsvorrichtung nicht zuverlässig erkannt. Ferner wird ein Altern sowohl des Endoskops als auch der Aufbereitungsvorrichtung erst bei Erreichen der Auslöseschwelle erkannt, so dass eine Aufbereitung dann nicht mehr möglich ist.

Die Trendanalyse der Dichtigkeitsprüfung über den Druckverlust bzw. die Druckverlustgeschwindigkeit ermöglicht es, beispielsweise durch Korrelation mit dem Endoskopindikator, festzustellen, ob ein sich über den Trendzeitraum veränderndes Verhalten der Aufbereitungsvorrichtung bzw. dessen Dichtigkeitsprüfer oder dem Endoskop zuordnen lässt. Ein Endoskop, das sich einem zu korrigierendem Ausmaß der Undichtigkeit nähert, kann dann zur Reparatur angezeigt werden. Über eine von den Endoskopen unabhängige Zunahme des Druckverlusts kann hingegen ein Problem des Dichtigkeitsprüfers der Aufbereitungsvorrichtung rechtzeitig erkannt und mitgeteilt werden, beispielsweise im Servicespeicher der Aufbereitungsvorrichtung für einen Servicetechniker hinterlegt werden.

Die der Erfindung zugrunde liegende Aufgabe wird auch durch ein System zur Überwachung einer Aufbereitungsvorrichtung für Endoskope, umfassend wenigstens eine Aufbereitungsvorrichtung und wenigstens eine Auswertungsvorrichtung, gelöst, bei dem die Auswertungsvorrichtung und die Auswertungsvorrichtung ausgebildet und eingerichtet sind, ein zuvor beschriebenes erfindungsgemäßes Verfahren durchzuführen.

Dabei kann die Auswertungsvorrichtung auch vorteilhaft in der Aufbereitungsvorrichtung integriert sein, diese also mittels der erfindungsgemäßen Trendanalyse eine Selbstdiagnose durchführen.

Die zum erfindungsgemäßen Verfahren und zum erfindungsgemäßen System genannten Merkmale, Eigenschaften und Vorteile gelten uneingeschränkt auch für die jeweils anderen Erfindungsgegenstände.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:

Fig. 1     eine schematische Darstellung eines erfindungsgemäßen Systems,

Fig. 2     eine grafische Darstellung einer erfindungsgemäßen Trendanalyse,

werten, aus der Trendanalyse eine IST-Kalibration zu errechnen und durch Vergleich mit einer Soll-Kalibration zu erkennen, ob eine Fehlkalibration vorliegt, wobei die Auswertungsvorrichtung weiter ausgebildet ist, als teilvorgangsrelevante Prozessparameter ein Teilvorgangsidentifikator, Dauer des Aufbereitungsteilvorgangs und/oder Dosiermengen eines oder mehrerer während des Teilvorgangs zu dosierender Aufbereitungsmittel zu protokollieren und in der Trendanalyse zu analysieren, ob sich mit der Zeit ein protokollierter Prozessparameter, insbesondere eine Dosiermenge oder eine Prozessdauer, so entwickelt, dass dieser Prozessparameter bei sich fortsetzendem Trend einen kalibrierten Bereich oder einen Toleranzbereich verlässt. Dabei kann die Auswertungsvorrichtung auch vorteilhaft in der Aufbereitungsvorrichtung integriert sein, diese also mittels der erfindungsgemäßen Trendanalyse eine Selbstdiagnose durchführen.

Besonders vorteilhaft ist das System ausgebildet und eingerichtet, ein zuvor beschriebenes erfindungsgemäßes Verfahren durchzuführen.

Die zum erfindungsgemäßen Verfahren und zum erfindungsgemäßen System genannten Merkmale, Eigenschaften und Vorteile gelten uneingeschränkt auch für die jeweils anderen Erfindungsgegenstände.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:

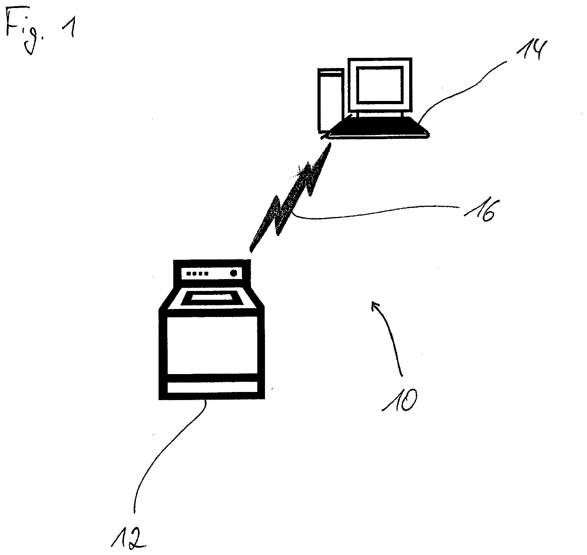

Fig. 1     eine schematische Darstellung eines erfindungsgemäßen Systems,

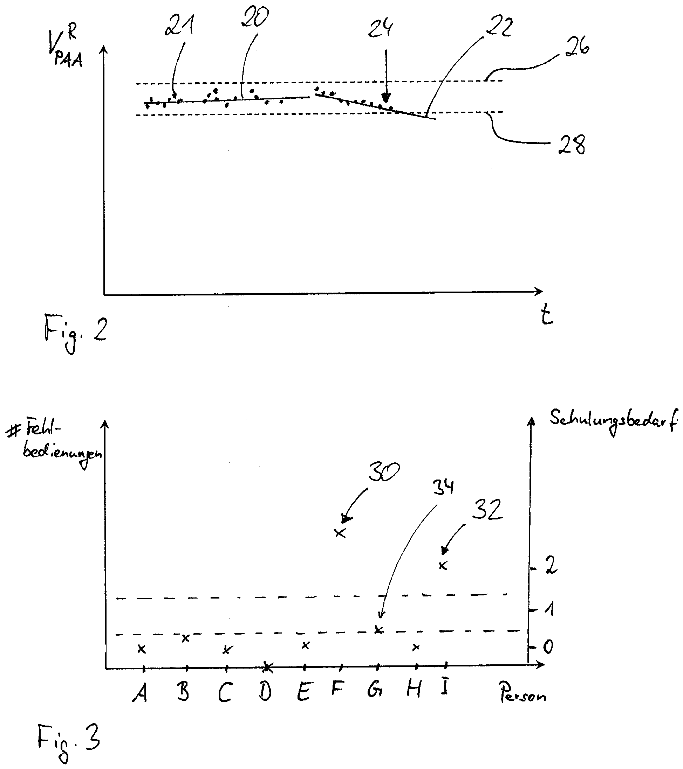

Fig. 2     eine grafische Darstellung einer erfindungsgemäßen Trendanalyse,

Fig. 3     eine grafische Darstellung einer weiteren erfindungsgemäßen Trendanalyse und

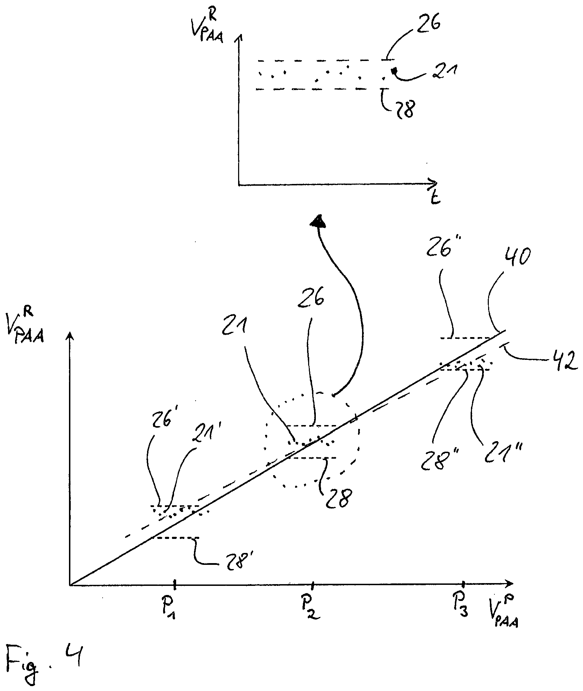

Fig. 4     eine grafische Darstellung einer weiteren erfindungsgemäßen Trendanalyse.

In den Zeichnungen sind jeweils gleiche oder gleichartige Elemente und/oder Teile mit denselben Bezugsziffern versehen, so dass von einer erneuten Vorstellung jeweils abgesehen wird.

In Fig. 1 ist ein erfindungsgemäßes System 10 zur Überwachung einer Aufbereitungsvorrichtung 12 für Endoskope dargestellt, das neben der Aufbereitungsvorrichtung 12 eine Auswertungsvorrichtung 14 umfasst. Die Aufbereitungsvorrichtung 12 und die Auswertungsvorrichtung 14 sind über eine Datenverbindung 16, beispielsweise eine ISDN-Verbindung oder LAN-Verbindung, miteinander verbunden. Auf diese Weise kann mittels der Auswertungsvorrichtung 14 eine Fernwartung der Aufbereitungsvorrichtung 12 erfolgen und die Aufbereitungsvorrichtung 12 kann Protokollierungsdaten an die Auswertungsvorrichtung 14 übermitteln.

Bei der Aufbereitungsvorrichtung 12 kann es sich beispielsweise um die automatische Aufbereitungsvorrichtung ETD3 der Anmelderin handeln. Alternativ kann die Auswertungsvorrichtung 14 auch in der Aufbereitungsvorrichtung 12 integriert sein. Dennoch kann auch in diesem Fall eine Datenverbindung 16 zu einer externen Auswertungsvorrichtung 14 vorhanden sein.

Die Aufbereitungsvorrichtung 12 dient dazu, Endoskope nach ihrer Verwendung zu reinigen und zu desinfizieren. Dabei durchläuft die Aufbereitungsvorrichtung 12 mehrere Aufbereitungsteilvorgänge, wie beispielsweise Vorwaschgang, Waschgang, Desinfiziergang, Spülgang und Trocknungsgang. Weitere Gänge können ebenfalls umfasst sein, wie beispielsweise Diagnosegänge für die Endoskope, z.B. ein Leck-Test.

Die Aufbereitungsvorrichtung 12 ist ausgebildet, mit ihrer Sensorik verschiedene Prozessparameter, wie beispielsweise die Identität der Bedienperson, die Identität der aufbereiteten Endoskope sowie Teilvorgangsdauern, Gesamtdauer, Mengen der eindosierten Aufbereitungsmittel, wie Wasser, Chemikalien, Waschmittel etc., zu protokollieren und dieses Protokoll an die Auswertungsvorrichtung 14 zu übermitteln. Die Auswertungsvorrichtung 14 ist ausgebildet, anhand dieser Daten über eine Mehrzahl von Aufbereitungsvorgängen eine Trendanalyse zu erstellen, um frühzeitig beispielsweise Wartungsbedarf für die Maschine oder Schulungsbedarf für Bedienpersonen identifizieren zu können.

Mehrere Beispiele möglicher Trendanalysen im Rahmen der Erfindung sind in den Figuren 2 bis 4 dargestellt.

Fig. 2 zeigt eine Trendanalyse eines Dosierparameters, nämlich den zeitlichen Verlauf über insgesamt 27 Aufbereitungsvorgänge der Dosierung von Peressigsäure (PAA). Die Datenpunkte 21 entsprechen jeweils einem Aufbereitungsvorgang. Der Parameter $V_{PAA}^{R}$ bezeichnet die real abgegebene PAA-Menge während eines Aufbereitungsvorgangs bzw. eines Desinfektions-Teilvorgangs eines Aufbereitungsvorgangs. Auf der x-Achse ist der Verlauf der Zeit eingetragen. Der zulässige Bereich für die Datenpunkte 21 ist durch eine Obergrenze 26 und durch eine Untergrenze 28 abgegrenzt. Messwerte außerhalb dieses Bereichs führen zu Fehlermeldungen und zu mangelhaften Desinfektionsergebnissen.

Die Trendanalyse ergibt, dass zunächst die Messwerte 21 sich in einem mittleren Bereich des zulässigen Bereichs befinden, ohne dass ein kritischer Trend erkennbar wäre. Der (lineare) Trend 20 ist in diesem Fall unkritisch. Etwa ab der Hälfte der Messzeit ergibt sich allerdings ein kritischer Trend 24, bei dem sich die einzelnen Messwerte 21 systematisch auf die Untergrenze 28 des zulässigen Bereichs zubewegen. Es wäre damit zu rechnen, dass bei weiteren Aufbereitungsvorgängen die tatsächlich abgegebene Dosiermenge von PPA unter die Untergrenze 28 absinken würde und zu mangelhaften Desinfizierungsergebnissen führen würde. Dieser Trend 22 ist zum Zeitpunkt 24 erkannt worden, so dass ein Wartungsbefehl oder eine Wartungsanfrage ausgegeben werden kann. Es wird dann kurzfristig eine Vor-Ort-Wartung oder eine Fernwartung durchgeführt, um das Problem, das zu dem kritischen Trend 22 führt, zu beheben. Damit wird es keine Aufbereitungsvorgänge geben, bei denen die abgegebene Menge von PAA unterhalb der Untergrenze 28 liegt.

In Fig. 3 ist eine andersartige Trendanalyse gezeigt, nämlich eine personenbezogene Trendanalyse. Hier wird für Personen A bis I aufgetragen, wie viele Fehlbedienungen oder was für eine Fehlbedienungsrate diese Bedienpersonen A bis I verursacht haben. Für die Personen A, B, C, D und H ist die Anzahl von Fehlbedienungen bzw. die Fehlbedienungsquote so gering, dass sie in den Bereich Null des Schulungsbedarfs fällt, so dass für diese Personen kein Schulungsbedarf besteht. Zwei Personen, nämlich F und I, weisen so hohe Anzahlen bzw. Raten von Fehlbedienungen 30, 32 auf, dass für sie ein hoher und dringender Schulungsbedarf der Kategorie 2 besteht. Diese Personen müssen zeitnah nachgeschult werden. Eine weitere Person G weist eine leicht erhöhte Anzahl bzw. Quote von Fehlbedienungen 34 auf, so dass bei dieser Person überlegt werden muss, ob bei ihr weiterer Schulungsbedarf vorhanden ist. Diese Person G fällt in den unteren Bereich des Bereichs 1 des Schulungsbedarfs.

Auch die Fehlbedienungsquote lässt sich in einer weiteren Trendanalyse für einzelne Personen gegenüber der Zeit darstellen, so dass gegebenenfalls festgestellt werden kann, wann für diese Personen aufgrund ihrer schlechter werdenden Fehlbedienungsquoten neue Schulungen angesetzt werden sollten. So lässt sich der Schulungsbedarf für einen gesamten Mitarbeiterstab koordinieren.

Anstelle von Fehlbedienungen kann auch die Anzahl von Fehlermeldungen und anderen möglicherweise personenbezogenen Indikatoren analysiert werden.

In Fig. 4 ist eine weitere erfindungsgemäße Trendanalyse grafisch dargestellt. Das untere, groß dargestellte, Koordinatensystem stellt die real abgegebene PAA-Menge $V_{PAA}^{R}$ gegen eine programmgerechte PAA-Menge $V_{PAA}^{P}$ dar. In diesem Fall sind auf der x-Achse, also der Achse für $V_{PAA}^{P}$ drei verschiedene Programme bzw. Teilprogramme $P_1$, $P_2$ und $P_3$ dargestellt, bei denen jeweils ein unterschiedlicher Mengenbedarf für PAA besteht, also jeweils unterschiedliche Dosiermengen abgegeben werden. Die erforderliche Dosiermenge im Programm $P_1$ ist niedrig, im Programm $P_2$ mittelhoch und im Programm $P_3$ hoch. Es kann sich auch um die Anzahl von ein, zwei oder drei aufzubereitenden Endoskopen in der Aufbereitungsvorrichtung 12 handeln, wodurch der Bedarf an PAA entsprechend skaliert. Mit dem Bezugszeichen 40 ist eine Soll-Kalibration bezeichnet, die die Sollwerte linear miteinander verbindet. Im idealen Fall bei korrekter Kalibration wäre somit die jeweils real abgegebene Menge $V_{PAA}^{R}$ gleich der Soll-Menge $V_{PAA}^{P}$ für die einzelnen Programme $P_1$, $P_2$ und $P_3$.

Zu den Programmen $P_1$, $P_2$ und $P_3$ sind jeweils Datenpunkte 21, 21' und 21" dargestellt. Wie im oberen Teil der Fig. 4 in einer Ausschnittsvergrößerung des mittleren Teils der unteren Grafik erkennbar ist, sind diese Datenpunkte 21, 21', 21" jeweils gegen eine Zeitachse aufgetragen, so dass die Kalibrierungs-Trendanalyse auch mit einer zeitlichen Trendanalyse kombinierbar ist.

Jede einzelne Datenfolge 21, 21', 21" entspricht also im Wesentlichen einer in Fig. 2 dargestellten zeitlichen Trendanalyse.

Wie sich aus Fig. 4 ergibt, liegt eine Fehlkalibrierung vor. Die Datenpunkte 21 zum mittleren Programm $P_2$ liegen mittig in dem zulässigen Bereich zwischen der Obergrenze 26 und der Untergrenze 28. Für das Programm $P_1$ mit geringem Bedarf an PAA liegen die Datenpunkte 21' allerdings in der Nähe der Obergrenze 26' des Bereichs zwischen Obergrenze 26' und Untergrenze 28'. Umgekehrt ergibt sich beim Programm $P_3$ mit einem hohen PAA-Bedarf eine Datenfolge mit Datenpunkten 21", die nahe der Untergrenze 28" des dort zutreffenden Bereichs zwischen Obergrenze 26" und Untergrenze 28" liegt. Es ergibt sich somit ein Trend zu einer systematischen Überdosierung bei kleinen Mengen und einer systematischen Unterdosierung bei großen Mengen. Dies wird durch eine interpolierte Ist-Kalibrationslinie 42 beschrieben, die eine geringere Steigung hat als die Soll-Kalibrationslinie 40. Die Datenpunkte 21, 21' und 21" können jeweils in einem Punkt bzw. einer Verteilung zusammengefasst werden, um die Kalibration richtig zu bestimmen. Es kann aber auch eine zeitliche Analyse der Veränderung der Kalibration erfolgen. Wenn eine solche Fehlkalibration durch die in Fig. 4 dargestellte Trendanalyse erkannt wird, können Maßnahmen getroffen werden, diese Fehlkalibrierung zu beheben, beispielsweise im Rahmen einer Fernwartung oder einer Vor-Ort-Wartung.

Alle genannten Merkmale, auch die den Zeichnungen allein zu entnehmenden sowie auch einzelne Merkmale, die in Kombination mit anderen Merkmalen offenbart sind, werden allein und in Kombination als offenbart angesehen. I

Bezugszeichenliste

| | |
|---|---|
| 10 | System |
| 12 | Aufbereitungsvorrichtung |
| 14 | Auswertungsvorrichtung |
| 16 | Datenverbindung |
| 20 | unkritischer Trend |
| 21 - 21" | Daten von Dosierungen |
| 22 | kritischer Trend |
| 24 | Zeitpunkt Wartungsbefehl |
| 26 - 26" | Obergrenze des zulässigen Bereichs |
| 28 - 28" | Untergrenze des zulässigen Bereichs |
| 30 | Anzahl Fehlbedienungen Person F |
| 32 | Anzahl Fehlbedienungen Person I |
| 34 | Anzahl Fehlbedienungen Person G |
| 40 | Soll-Kalibration |
| 42 | Ist-Kalibration |
| $t$ | Zeit |
| $V_{PAA}^{R}$ | real abgegebene PAA-Menge |

$$V_{PAA}^{P}$$ programmgerechte PAA-Menge

**Patentansprüche**

1. Verfahren zur Überwachung einer Aufbereitungsvorrichtung (12) für Endoskope, insbesondere einer Reinigungs- und/oder Desinfiziervorrichtung, wobei über eine Mehrzahl von Aufbereitungsvorgängen, die jeweils mehrere Teilvorgänge (P1, P2, P3) umfassen, von wenigstens einem Endoskop in einer einzigen Aufbereitungsvorrichtung (12) mehrere Datenpunkte (21, 21', 21") eines Prozessparameters ($V_{PAA}$) protokolliert werden, wobei bei jedem Teilvorgang (P1, P2, P3) ein unterschiedlicher Bedarf des Prozessparameters ($V_{PAA}$) besteht, wobei in jedem Datenpunkt (21, 21', 21") des Prozessparameters ($V_{PAA}$) ein Zeitpunkt (t) des jeweiligen Aufbereitungsvorgangs protokolliert und in Zuordnung zum jeweiligen Teilvorgang (P1, P2, P3) gespeichert wird, wobei in der Auswertungsvorrichtung (14) über die Teilvorgänge (P1, P2, P3) eine Trendanalyse der mehreren Datenpunkte (21, 21', 21") durchgeführt wird, wobei aus der Trendanalyse eine IST-Kalibration (42) des Prozessparameters ($V_{PAA}$) errechnet wird und durch Vergleich mit einer Soll-Kalibration (40) erkannt wird, ob eine Fehlkalibration der Aufbereitungsvorrichtung (12) vorliegt.

2. Verfahren nach Anspruch 1, wobei als teilvorgangsrelevante Prozessparameter ein Teilvorgangsidentifikator, Dauer des Teilvorgangs (P1, P2, P3) und/oder Dosiermengen eines oder mehrerer während des Teilvorgangs (P1, P2, P3) zu dosierender Aufbereitungsmittel protokolliert werden, wobei in der Trendanalyse analysiert wird, ob sich mit der Zeit ein protokollierter Prozessparameter ($V_{PAA}$), insbesondere eine Dosiermenge oder eine Prozessdauer, so entwickelt, dass dieser Prozessparameter ($V_{PAA}$) bei sich fortsetzendem Trend einen kalibrierten Bereich oder einen Toleranzbereich verlässt.

3. Verfahren nach Anspruch 1 oder 2, wobei zusätzlich zum teilvorgangsrelevanten Prozessparameter ein Aufbereitungsvorrichtungsindikator, ein Bedienpersonenindikator, ein Aufbereitungsprogrammindikator, wenigstens ein Endoskopindikator für wenigstens ein aufbereitetes Endoskop, wenigstens ein Aufbereitungsmittelindikator, ein Druckverlust und/oder eine Druckverlustgeschwindigkeit, wenigstens eine Dosiermenge wenigstens eines zu dosierenden Aufbereitungsmittels, ein Start- und/oder Endzeitpunkt des Aufbereitungsvorgangs, eine Prozessdauer, Fehlfunktionen, Fehlbedienungen und/oder Fehlermeldungen protokolliert wird oder werden.

4. Verfahren nach einem der Ansprüche 1 bis 3, wobei in der Trendanalyse wenigstens ein protokollierter Prozessparameter ($V_{PAA}$) als Funktion der jeweiligen Start- oder Endzeitpunkte der Aufbereitungsvorgänge, des Aufbereitungsvorrichtungsindikators, des Aufbereitungsprogrammindikators, des Teilvorgangsindikators, des Bedienpersonenindikators und/oder des Endoskopindikators dargestellt und/oder ausgewertet wird.

5. Verfahren nach einem der Ansprüche 1 bis 4, wobei die protokollierten Datenpunkte (21, 21', 21") für die Darstellung und/oder Auswertung nach dem Zeitpunkt, dem Aufbereitungsvorrichtungsindikator, dem Bedienpersonenindikator, dem Aufbereitungsprogrammindikator, dem Teilprogrammindikator und/oder dem wenigstens einen Endoskopindikator gefiltert werden.

6. Verfahren nach einem der Ansprüche 1 bis 5, wobei ein Wartungsbedarf (24) der Aufbereitungsvorrichtung (12) signalisiert wird, bevor ein protokollierter Prozessparameter ($V_{PAA}$) den kalibrierten Bereich oder Toleranzbereich verlässt, wenn die Trendanalyse einen solchen Trend (22) ergibt.

7. Verfahren nach einem der Ansprüche 1 bis 6, wobei ein Schulungsbedarf einer Bedienperson signalisiert wird, wenn die Trendanalyse ergibt, dass bei der Bedienperson (30, 32) die Häufigkeit des Auftretens von Fehlfunktionen, Fehlbedienungen und/oder Fehlermeldungen gegenüber anderen Bedienpersonen erhöht ist.

8. System (10) zur Überwachung einer Aufbereitungsvorrichtung (12) für Endoskope, umfassend wenigstens eine Aufbereitungsvorrichtung (12) und wenigstens eine Auswertungsvorrichtung (14), bei dem die Aufbereitungsvorrichtung (12) und die Auswertungsvorrichtung ausgebildet und eingerichtet sind, ein Verfahren nach einem der Ansprüche 1 bis 7 durchzuführen.

9. System (10) nach Anspruch 8, wobei die Auswertungsvorrichtung (14) in der Aufbereitungsvorrichtung (12) integriert ist.

**Claims**

1. A method for monitoring a reprocessing device (12) for endoscopes, in particular a cleaning and/or disinfection device, wherein a plurality of data points (21, 21', 21") of a process parameter ($V_{PAA}$) of at least one endoscope in a single reprocessing device (12) are logged over a plurality of reprocessing operations which each comprise several different partial operations (P1, P2, P3), wherein in each partial operation (P1, P2, P3) the need for the process parameter ($V_{PAA}$) is different, wherein in each data point (21, 21', 21") of the process parameter (21, 21', 21") a time (t) of each reprocessing operation is logged and stored in relation to the respective partial operation (P1, P2, P3), wherein a trend analysis of the plurality of data points (21, 21', 21") is performed in the evaluation device (14) over the partial operations (P1, P2, P3), wherein an actual calibration (42) of the process parameter ($V_{PAA}$) is calculated from the trend analysis and from a comparison with a target calibration (40) it is determined whether there is an incorrect calibration of the reprocessing device (12).

2. The method according to claim 1, wherein as partial-operation-relevant process parameter a partial operation identifier, a duration of the partial operation (P1, P2, P3) and/or dose quantities of one or more reprocessing agents to be metered during the partial operation (P1, P2, P3) is logged, wherein it is analyzed in the trend analysis whether, with time, a logged process parameter ($V_{PAA}$), in particular a dose quantity or a process duration, develops such that this process parameter leaves a calibrated range or a tolerance range if the trend continues.

3. The method according to claim 1 or 2, wherein in addition to the partial-operation-relevant process parameter, a reprocessing device indicator, an operator indicator, a reprocessing program indicator, at least one endoscope indicator for at least one reprocessed endoscope, at least one reprocessing agent indicator, a pressure loss and/or a pressure loss speed, at least one dose quantity of at least one reprocessing agent to be metered, a start and/or end time of the reprocessing operation, a process duration, malfunctions, operating errors and/or error messages is or are logged.

4. The method according to one of claims 1 to 3, wherein in the trend analysis, at least one logged process parameter ($V_{PAA}$) is preferably represented and/or evaluated as a function of the respective start or end times of the reprocessing operations, of the reprocessing device indicator, of the reprocessing program indicator, of the partial operation indicator, of the operator indicator and/or of the endoscope indicator.

5. The method according to one of claims 1 to 4, wherein the logged data points (21, 21', 21") for the representation and/or evaluation are filtered by the point in time, the reprocessing device indicator, the operator indicator, the reprocessing program indicator, the partial program indicator and/or the at least one endoscope indicator.

6. The method according to one of claims 1 to 5, wherein in a servicing need (24) of the reprocessing device (12) is signaled before a logged process parameter ($V_{PAA}$) has left the calibrated range or tolerance range if the trend analysis indicates such a trend (22).

7. The method according to one of claims 1 to 6, wherein a need for training for an operator is signaled if the trend analysis shows that the frequency of malfunctions, operating errors and/or error messages is elevated for one operator (30, 32) compared to other operators.

8. A system (10) for monitoring a reprocessing device (12) for endoscopes, comprising at least one reprocessing device (12) and at least one evaluation device (14), in which the reprocessing device (12) is designed and set up to perform a method according to one of claims 1 to 7.

9. The system (10) according to claim 8, wherein the evaluation device (14) is integrated in the reprocessing device (12).

## Revendications

1. Procédé de surveillance d'un dispositif (12) de retraitement pour endoscopes, en particulier un dispositif de nettoyage et/ou de désinfection, dans lequel, par une pluralité d'opérations de retraitement, dont chacune comprend plusieurs sous-processus (P1, P2, P3), une pluralité de points de données (21, 21', 21") d'un paramètre de processus ($V_{PAA}$) est enregistrée par au moins un endoscope dans un seul dispositif de retraitement (12), un besoin différent existant pour le paramètre de processus (VPAA) pour chaque sous-processus (P1, P2, P3), un temps (t) du processus de retraitement respectif étant enregistré pour chaque point de données (21, 21', 21") du paramètre de processus ($V_{PAA}$) et étant mémorisé en association avec le sous-processus respectif (P1, P2, P3), une analyse de tendance de ladite pluralité de points de données (21, 21', 21") du sous-processus (P1, P2, P3) étant effectuée dans le dispositif d'évaluation (14) par l'intermédiaire des sous-processus (P1, P2, P3), un étalonnage effectif (42) du paramètre de processus ($V_{PAA}$) étant calculé à partir de l'analyse de tendance et permettant d'identifier, par comparaison avec un étalonnage nominal (40), s'il existe un étalonnage incorrect du dispositif de retraitement (12).

2. Procédé selon la revendication 1, dans lequel un identificateur de sous-processus, la durée du sous-processus (P1, P2, P3) et/ou des quantités de dosage d'un ou plusieurs agents de traitement à doser pendant le sous-processus (P1, P2, P3) sont enregistrés en tant que paramètres de processus pertinents pour le sous-processus, l'analyse de tendance permettant de déterminer si un paramètre de processus enregistré ($V_{PAA}$), en particulier une quantité de dosage ou une durée de processus, évolue dans le temps de telle sorte que ce paramètre de processus ($V_{PAA}$) quitte une plage calibrée ou une plage de tolérance si la tendance se poursuit.

3. Procédé selon la revendication 1 ou la revendication 2, dans lequel, outre le paramètre de processus pertinent pour le sous-processus, un indicateur de dispositif de retraitement, un indicateur de personnel d'exploitation, un indicateur de programme de retraitement, au moins un indicateur d'endoscope pour au moins un endoscope retraité, au moins un indicateur d'agent de retraitement, une perte de pression et/ou une vitesse de perte de pression, au moins une quantité de dosage d'au moins un agent de retraitement à doser, un point temporel de début et/ou de fin du processus de retraitement, une durée de processus, des dysfonctionnements, des erreurs de fonctionnement et/ou des messages d'erreur sont enregistrés.

4. Procédé selon l'une des revendications 1 à 3, dans lequel, dans l'analyse de tendance, au moins un paramètre de processus enregistré ($V_{PAA}$) est affiché et / ou évalué en fonction des points temporels de début ou de fin respectives des processus de retraitement, de l'indicateur du dispositif de retraitement, de l'indicateur du programme de retraitement, de l'indicateur de sous-processus, de l'indicateur de la personne opératrice et/ou de l'indicateur de l'endoscope.

5. Procédé selon l'une des revendications 1 à 4, dans lequel les points de données enregistrés (21, 21', 21") pour l'affichage et / ou l'évaluation sont filtrés en fonction du temps, de l'indicateur du dispositif de retraitement, de l'indicateur de la personne opératrice, de l'indicateur du programme de retraitement, de l'indicateur du programme partiel et/ou d'au moins un indicateur d'endoscope.

6. Procédé selon l'une des revendications 1 à 5, dans lequel un besoin de maintenance (24) du dispositif de retraitement (12) est signalé avant qu'un paramètre de processus enregistré ($V_{PAA}$) ne quitte la plage calibrée ou la plage de tolérance, si l'analyse de tendance montre une telle tendance (22).

7. Procédé selon l'une des revendications 1 à 6, dans lequel un besoin de formation d'une personne opératrice est signalé si l'analyse de tendance montre que la fréquence d'occurrence des dysfonctionnements, des erreurs d'exploitation et/ou des messages d'erreur est accrue pour la personne opératrice (30, 32) par rapport aux autres personnes opératrices.

8. Système (10) de contrôle d'un dispositif de retraitement (12) pour endoscopes, comprenant au moins un dispositif de retraitement (12) et au moins un dispositif d'évaluation (14), le dispositif de retraitement (12) et le dispositif d'évaluation étant conçus et réglés pour mettre en œuvre un procédé selon l'une des revendications 1 à 7.

9. Le système (10) selon la revendication 8, dans lequel le dispositif d'évaluation (14) est intégré dans le dispositif de traitement (12).

Fig. 1

14

16

10

12

$V_{PAA}^{R}$

21    20         24      22        26

28

Fig. 2

t

# Fehl-
bedienungen

Schulungsbedarf

30

34    32

× 30

× 32
2

34
× ×

1

× × × × × × ×
0

A   B   C   D   E   F   G   H   I   Person

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1529484 A2
- US 5422267 A
- US 20100071736 A1